# EUROPÄISCHE PATENTSCHRIFT

(11) **EP 3 448 358 B1**
(45) Veröffentlichungstag und Bekanntmachung des Hinweises auf die Patenterteilung: **08.09.2021**
(21) Anmeldenummer: 17718895.0
(22) Anmeldetag: 20.04.2017
(51) Int. Cl.: A61K 9/00, A61K 47/38, A61K 38/28, A61K 38/26, A61P 3/10

(54) **FILMFÖRMIGE DARREICHUNGSFORM ZUR TRANSMUKOSALEN VERABREICHUNG VON PEPTID-ANTIDIABETIKA**
FILM ADMINISTRATION FORM FOR THE TRANS-MUCOSAL ADMINISTRATION OF PEPTIDE ANTI-DIABETIC DRUGS
FORME D'ENRICHISSEMENT SOUS FORME DE FILM DESTINEE A L'ADMINISTRATION TRANSMUQUEUSE D'ANTI-DIABETIQUES DE PEPTIDE

(30) Priorität: 26.04.2016 EP 16167053
(43) Veröffentlichungstag der Anmeldung: 06.03.2019
(73) Patentinhaber: LTS Lohmann Therapie-Systeme AG, 56626 Andernach (DE)
(72) Erfinder: MÜLLER, Markus, 53840 Troisdorf (DE); HAMMES, Claudia Maria, 56626 Andernach (DE); SCHMITZ, Christoph, 56598 Rheinbrohl (DE); LINN, Michael, 55596 Waldböckelheim (DE); SAMETI, Mohammad, 53177 Bonn (DE); KLAFFENBACH, Peter, 41564 Kaarst (DE)
(74) Vertreter: Held, Stephan
(86) Internationale Anmeldenummer: PCT/EP2017/059379
(87) Internationale Veröffentlichungsnummer: WO 2017/186563

(56) Entgegenhaltungen:
- WO-A1-98/17251
- WO-A1-2011/156711
- WO-A1-2012/104834

## Beschreibung

Die vorliegende Erfindung betrifft filmförmige Darreichungsformen, insbesondere filmförmige Darreichungsformen zur Verabreichung eines Peptid-Antidiabetikums über eine Schleimhaut.

Die Zuckerkrankheit (Diabetes mellitus) ist eine Stoffwechselerkrankung, die durch chronisch erhöhte Blutzuckerwerte gekennzeichnet ist. Die Zuckerkrankheit wird durch einen Mangel am Hormon Insulin (Typ-1 Diabetes) oder durch eine verminderte Empfindlichkeit der Körperzellen auf die Wirkung von Insulin (Typ-2 Diabetes) verursacht.

Insulin ist für die Steuerung der Zuckerkonzentration im Blut (Glukosespiegel) wichtig und es ist das einzige Hormon im Körper, das den Glukosespiegel senken kann. Ein chronisch erhöhter Blutzucker verursacht bei Diabetespatienten tiefgreifende Folgeschäden zu denen eine Schädigung der Netzhaut (diabetische Retinopathie), Empfindungsstörungen der Nerven (Neuropathie) sowie Ketoazidose (Übersäuerung durch mangelnde Glucoseverwertung) gehören. Es kann aber aufgrund eines zu niedrigen Blutzuckerspiegels ("unterzuckert") auch zu einer Bewusstlosigkeit (diabetischer Schock) kommen. Für die Gesundheit der Patienten ist es daher sehr wichtig, einen ausgeglichenen Blutzuckerspiegel aufrecht zu erhalten. Dies kann durch eine Verabreichung von Insulin angestrebt werden.

Weil Insulin bei peroraler Verabreichung von proteolytischen Enzymen im Verdauungstrakt rasch abgebaut wird, erfolgt die Verabreichung von Insulin, aber auch von Insulin-Analoga sowie von anderen Peptid-Antidiabetika, bei Patienten mit Diabetes mellitus üblicherweise mittels Injektionen. Diese Art der Verabreichung wird von vielen Patienten jedoch als große Beeinträchtigung ihrer Lebensqualität empfunden, nicht zuletzt wegen Missbehagen und/oder Ängsten vor den mit der Injektion verbundenen Schmerzen oder einem Unbehagen durch die für die Injektion erforderliche Entblößung.

Ungeachtet der Probleme, die einer oralen Verabreichung von Insulin entgegenstehen, wurden Anstrengungen unternommen, perorale Darreichungsformen für Insulin zu entwickeln. Ein Überblick über diese Bemühungen gibt der Übersichtsartikel von L. Heinemann und Y. Jaques (Heinemann, L. and Jaques, Y. (2009) Oral Insulin and Buccal Insulin: A Critical Reappraisal. Journal of Diabetes Science and Technology 3, Seiten 568-584).

Für eine orale Verabreichung von Insulin kommen grundsätzlich auch filmförmige Darreichungsformen in Betracht, mit denen Insulin über die Mundschleimhaut verabreicht werden kann. Beispielsweise offenbart die WO 2012/104834 A1 an Schleimhaut haftende, filmförmige Darreichungsformen umfassend eine erste Polymerzubereitung, die wasserlösliche Polymere aufweist, die ein Auflösen und Freisetzen des Wirkstoffs innerhalb von 20 Minuten oder weniger ermöglicht, eine zweite Polymerzubereitung, umfassend hydrophile, bioadhäsive Polymere, die eine andauernde Freisetzung des Wirkstoffs über einen Zeitraum von etwa 1 Stunde bis etwa 20 Stunden ermöglicht, und Insulin oder ein Insulinanalog als Wirkstoff, wobei die Darreichungsform nicht dicker als 0,5 mm ist. In dieser Druckschrift konkret beschriebene filmförmige Darreichungsformen waren zumindest in *in-vitro* Versuchen mit einem bukkalen Zellmodell in der Lage, Insulin freizusetzen. In vivo Studien wurden jedoch nicht veröffentlicht. In der Offenlegungsschrift WO 2009/048945 A1 werden filmförmige Zubereitungen zur systemischen Verabreichung von Insulin offenbart, die an einer Schleimhaut haften können. Die Filme sollen sich in Abhängigkeit ihrer Hilfsstoffe in einem Zeitraum von weniger als 30 Sekunden bis zu einer halben Stunde auflösen oder das Insulin freisetzen. Die Zubereitungen enthalten humanes Insulin, einen Zinkchelator und ein Auflösungsmittel. Sofern es sich bei den filmförmigen Zubereitungen um mehrschichtige Filme handelt, liegen Insulin einerseits und der Zinkchelator und/oder das Auflösungsmittel andererseits in separaten Schichten vor, so dass der Wirkstoff von den genannten Hilfsstoffen getrennt gelagert wird, um die Haltbarkeit des Wirkstoffs zu verbessern.

Die unter der Veröffentlichungsnummer WO 98/17251 A1 veröffentlichte internationale Patentanmeldung betrifft filmförmige Träger für die Verabreichung pharmazeutischer Wirkstoffe an Schleimhautoberflächen. Diese Zubereitungen sollen für die Verabreichung einer Vielzahl von Wirkstoffen, einschließlich Insulin, geeignet sein.

Die WO 2011/156711 A1 offenbart filmförmige Darreichungsformen auf Basis von mindestens einem Polymer, z.B. Hydroxylpropylmethylcellulose, die Nanopartikel umfassen, zur Verwendung in der Medizin, insbesondere zur Behandlung von Störungen der Blutzuckerregulation.

Vor dem Hintergrund des vorgenannten Standes der Technik, lag der vorliegenden Erfindung die Aufgabe zugrunde, eine filmförmige Darreichungsform bereitzustellen, mit der Peptid-Antidiabetika über eine Schleimhaut des Mundraums verabreicht werden können, wobei die Verweildauer der Zubereitung auf der Mundschleimhaut mindestens 30 Minuten bis eine Stunde oder noch länger betragen soll, um in diesem Zeitraum eine therapeutisch wirksame Menge des Peptid-Antidiabetikums über die Mundschleimhaut an den Blutkreislauf eines Patienten verabreichen zu können. Hierbei war zu berücksichtigen, dass eine Applikation der filmförmigen Darreichungsform auf einer Mundschleimhaut nicht zu einem für den Anwender inakzeptablen Mundgefühl führen soll. Gleichzeitig sollte die Darreichungsform kontinuierlich herstellbar sein, um eine möglichst effiziente und somit kostengünstige Herstellung zu ermöglichen.

Die Aufgabe wird überraschenderweise durch eine filmförmige Darreichungsform gelöst, die mindestens eine wirkstoffhaltige Schicht umfasst, bei der ein Peptid-Antidiabetikum als Wirkstoff in einer Matrix auf Basis von mindestens zwei Hydroxypropylmethylcellulosen enthalten ist, die sich in ihrer Viskosität voneinander unterscheiden.

Gemäß einem ersten Aspekt betrifft die Erfindung eine filmförmige Darreichungsform zur Verabreichung mindestens eines Peptid-Antidiabetikums über eine Mundschleimhaut.

Gemäß einem zweiten Aspekt betrifft die Erfindung ein Verfahren zur Herstellung einer filmförmigen Darreichungsform zur Verabreichung mindestens eines Peptid-Antidiabetikums über eine Mundschleimhaut.

Gemäß einem dritten Aspekt betrifft die Erfindung die Verwendung einer filmförmigen Darreichungsform zur Verabreichung mindestens eines Peptid-Antidiabetikums über eine Mundschleimhaut.

Gemäß einem weiteren Aspekt umfasst die Erfindung ein Verfahren zur Verabreichung mindestens eines Peptid-Antidiabetikums über die Mundschleimhaut.

Gemäß noch einem weiteren Aspekt umfasst die Erfindung Verfahren zur Behandlung von Patienten, die der Verabreichung eines Peptid-Antidiabetikums bedürfen.
Fig. 1 zeigt eine Graphik, welche die Permeation von Insulin bzw. einem Insulin-Analogon aus zwei unterschiedlichen filmförmigen Darreichungsformen in einem *in vitro*-Permeationsassay veranschaulicht.
Fig. 2 zeigt eine Graphik, welche die Permeation eines Insulin-Analogons aus drei unterschiedlichen filmförmigen Darreichungsformen in einem *in vitro*-Permeationsassay veranschaulicht.
Fig. 3 zeigt eine Graphik, welche die Permeation eines Insulin-Analogons aus einer filmförmigen Darreichungsform in einem *in vitro*-Permeationsassay veranschaulicht.
Fig. 4 zeigt eine Graphik, welche die Permeation eines Insulin-Analogons aus einer filmförmigen Darreichungsform in einem *in vitro*-Permeationsassay veranschaulicht.
Fig. 5 zeigt eine Graphik, welche die Permeation eines Insulin-Analogons aus einer filmförmigen Darreichungsform in einem *in vitro*-Permeationsassay veranschaulicht.

Die filmförmige Darreichungsform gemäß dem ersten Aspekt umfasst mindestens eine wirkstoffhaltige Schicht, die als Wirkstoff mindestens ein Peptid-Antidiabetikum enthält, und die bei korrekter Applikation der Darreichungsform mit der Mundschleimhaut in Kontakt kommt, um den in ihr enthaltenen Wirkstoff über die Mundschleimhaut abzugeben.

Die mindestens eine wirkstoffhaltige Schicht weist einen Gehalt an mindestens einem Peptid-Antidiabetikum auf. Die mindestens eine wirkstoffhaltige Schicht kommt bei Applikation der filmförmigen Darreichungsform mit der Mundschleimhaut in Kontakt und haftet an der Mundschleimhaut. Das in der mindestens einen wirkstoffhaltigen Schicht enthaltene Peptid-Antidiabetikum wird aus der mindestens einen wirkstoffhaltigen Schicht über die Mundschleimhaut an den Blutkreislauf des Anwenders abgegeben.

Der Begriff "Peptid-Antidiabetikum" im Sinne der vorliegenden Offenbarung bezeichnet als Antidiabetikum, also als Wirkstoff zur Behandlung eines Diabetes mellitus, wirksame Peptide. Der Begriff "Peptid-Antidiabetikum" umfasst Insuline und Insulin-Analoga, aber auch Inkretine und Inkretin-Mimetika.

Bei dem Insulin handelt es sich vorzugsweise um humanes Insulin, es kann aber auch Schweineinsulin oder Rinderinsulin verwendet werden. Die Insulin-Analoga umfassen kurz und rasch wirkende Insulinanaloga wie Insulin aspart, Insulin lispro, und Insulin glulisin, aber auch langwirkende Insulinanaloga wie Insulin detemir, Insulin glargin, Insulin degludec und Isophan-Insulin.

Insulin ist ein für alle Wirbeltiere lebenswichtiges Proteohormon, das in den β-Zellen der Bauchspeicheldrüse gebildet wird. Insulin besteht aus zwei Peptidketten, der A-Kette mit 21 Aminosäuren und der B-Kette mit 30 Aminosäuren, welche durch zwei intermolekulare Disulfidbrücken kovalent verbunden sind (Cys-A7 mit Cys-B7 und Cys-A20 mit Cys-B19). Eine dritte intramolekulare Disulfidbrücke verbindet die Cysteinreste der Positionen 6 und 11 der A-Kette.

Natürlicherweise wird Insulin als Präproinsulin synthetisiert. Präproinsulin ist eine Polypeptid-Molekül mit 110 Aminosäuren, bestehend aus einer 24 Aminosäure langen Signalsequenz, 2 Aminosäure-Resten, eine sich daran anschließende, 30 Aminosäure lange B-Kette, eine darauf folgendes 31 Aminosäure langes C-Peptid, gefolgt von zwei weiteren Aminosäure Resten und der A-Kette mit 21 Aminosäuren.

Durch Bildung von drei Disulfidbrücken, zwei zwischen dem A- und dem B-Peptid, und einer innerhalb des A-Peptids, wird das Präproinsulin gefaltet. Die Signalsequenz und das C-Peptid werden von dem gefalteten Präpropeptid abgespalten. Die Signalsequenz wird beim Durchtritt des Präproinsulins durch die Membran des ER abgespalten, wodurch das Proinsulin entsteht. Das Proinsulin wird in den Golgi-Apparat aufgenommen und dort gespeichert. Bei Bedarf wird die C-Kette durch spezifische Proteasen abgespalten, so dass das resultierende Insulin seine endgültige Struktur erreicht.

Schweineinsulin ist die gereinigte, natürliche, antidiabetisch wirkende Substanz aus der Bauchspeicheldrüse des Schweins. Es hat dieselbe Struktur wie Humaninsulin bis auf die Position B30 der B-Kette, wo es ein Alanin statt ein Threonin aufweist.

Insulin vom Rind (C₂₅₄H₃₇₇N₆₅O₇₅S₆, Mᵣ = 5734 g/mol) ist eine gereinigte, natürliche, antidiabetisch wirkende Substanz aus Rinderpankreas. Rinderinsulin unterscheidet sich in drei Aminosäuren von Humaninsulin.

Die Primärstruktur von Insulin aspart (C₂₅₆H₃₈₁N₆₅O₇₉S₆, Mᵣ = 5825.8 g/mol) ist mit Ausnahme von Asparaginsäure (= Aspartinsäure, deshalb "aspart") an Stelle von Prolin in Position 28 der B-Kette identisch mit der Primärstruktur von Humaninsulin. Der Ersatz der Aminosäure führt nach der subkutanen Verabreichung zu einer besseren Absorption in Blut und zu einer schnelleren Elimination.

Insulin lispro (C₂₅₇H₃₈₃N₆₅O₇₇S₆, Mᵣ = 5808 g/mol) hat eine identische Primärstruktur wie humanes Insulin, mit Ausnahme der Aminosäuren, die in Position 28 und 29 der B-Kette gegeneinander vertauscht sind.

Die Primärstruktur von Insulin glulisin (C₂₅₈H₃₈₄N₆₄O₇₈S₆, Mᵣ = 5823 g/mol) ist identisch mit der Primärstruktur von Humaninsulin, mit folgenden Ausnahmen:
Glutaminsäure statt Lysin an der Position 29 der B-Kette Lysin statt Asparagin an der Position 3 der B-Kette.

Insulin detemir (C₂₆₇H₄₀₂O₇₆N₆₄S₆, Mᵣ = 5916.9 g/mol) hat eine identische Primärsequenz wie humanes Insulin mit Ausnahme des entfernten Threonins in Position B30 der B-Kette und einem zugefügten Molekül Myristinsäure an das Lysin in Position B29.

Insulin glargin (C₂₆₇H₄₆₄N₇₂O₇₈S₆, Mᵣ = 6063 g/mol) hat dieselbe Primärstruktur wie humanes Insulin, mit folgenden Ausnahmen:
A-Kette: Glycin anstelle von Asparagin an Position 21
B-Kette: Zusätzlich zwei Arginine an Position 31 und 32.

Insulin degludec hat im Wesentlichen dieselbe Struktur wie Humaninsulin, mit den folgenden Ausnahmen:
die letzte Aminosäure der B-Kette (Threonin B30) wurde entfernt;
an das Lysin B29 wurde eine Glutaminsäure und eine 16-C-Fettsäure angehängt.

Bei Isophan-Insulinen (= NPH-Insuline) handelt es sich um Insuline, die mit Protaminsulfat oder einem anderen geeigneten Protamin komplexiert sind. Protamine sind basische Peptide, die aus Sperma oder Eiern von Fischen durch Extraktion gewonnen werden (meist Lachsfische oder Heringe). Isophan-Insulin hat einen späteren Wirkungseintritt und eine längere Wirkdauer als gewöhnliches Insulin und gehört zu den Verzögerungsinsulinen.

Zu den Inkretinen gehören die gastrointestinalen Hormone, die die nahrungsabhängige Sekretion von Insulin aus Betazellen des Pankreas steuern. Zu den Inkretinen zählen das Glucagon-ähnliche Peptid (Glucagon-like peptide 1; GLP-1) und das Glucose abhängige insulinotrope Peptid (Glucose dependent insulinotropic peptide; GIP).

Das Glucagon-ähnliche Peptid 1 ist ein Polypeptid, dessen Aminosäuresequenz durch das Proglucagon-Gen bestimmt wird. Das Glucagon-ähnliche Peptid kann in zwei unterschiedlichen biologisch aktiven Formen vorliegen, dem GLP-1-(7-37) mit einer Primärstruktur aus 30 Aminosäuren und dem GLP-1-(7-36) mit einer Primärstruktur aus 31 Aminosäuren. Neben anderen Wirkungen stimuliert das Glucagon-ähnliche Peptid 1 die Insulinsynthese in der Bauchspeicheldrüse, es senkt den Glucagonspiegel, verzögert die Magenentleerung und hemmt das Hunger- und Durstgefühl.

Das Glucose abhängige insulinotrope Peptid wird eigentlich in den K-Zellen des Duodenums und des Jejunums gebildet. Es handelt sich um ein Polypeptid, dessen Primärstruktur aus 42 Aminosäuren besteht. GIP fördert die Freisetzung von Insulin aus den B-Zellen des Pankreas. Es hemmt die Magenmotorik und die Sekretion von Magensaft.

Inkretin-Mimetika sind blutzuckersenkende und antidiabetische Wirkstoffe, deren Wirkung den körpereignen Inkretinen entsprechen. Inkretin-Mimetika werden auch als GLP-1-Agonisten, GLP-1-Analoga oder GLP-1-Rezeptor-Agonisten bezeichnet. Zu den für eine transmukosale Verabreichung in Frage kommenden Inkretin-Mimetika gehören Exenatid, Liraglutid und Lixisenatid.

Exenatid ist ein synthetisches Peptid, das aus 39 Aminosäuren besteht. Die Substanz stammt ursprünglich aus dem Gift der Gila-Krustenechse (Gila-Monster, *Heloderma suspectum*)*.* Das natürliche Peptid wird als Exendin-4 bezeichnet. Exenatid ist mit dem Inkretin Glucagon-like Peptide-1 (GLP-1) verwandt und hat eine Sequenzübereinstimmung von 53%. Exenatid hat eine wesentlich längere Halbwertszeit und Wirkdauer als GLP-1, weil es nicht von der Dipeptidylpeptidase-4 (DPP-4) abgebaut wird.

Liraglutid oder γ-L-Glutamoyl(N-α-hexadecanoyl)-Lys²⁶, Arg³⁴-GLP-1(7-37) ist ein verzweigtkettiges Peptid. Es ist ein Analogon des Inkretins GLP-1. Die Sequenzübereinstimmmung liegt bei 97%. Lys 34 wurde durch Arg ersetzt und an Lys 26 wurde über einen Glu-Spacer eine C16-Fettsäure angehängt. Durch diese Modifikationen konnte die Halbwertszeit von GLP-1 (2 Minuten) auf 13 Stunden verlängert werden.

Lixisenatid ist ein Peptid aus 44 Aminosäuren und ein GLP-1-Analog, das wie Exenatid ausgehend von Exendin-4 entwickelt wurde. Lixisenatid hat eine längere Halbwertszeit und eine höhere Bindungsaffinität als das natürliche Substrat GLP-1. Es hat jedoch eine kürzere Halbwertszeit als Liraglutid.

Gemäß einer zusätzlichen und/oder alternativen Ausführungsform ist das mindestens eine Peptid-Antidiabetikum in einer Menge von mindestens 5 Gew.-%, insbesondere mindestens 5,6 Gew.-%, vorzugsweise mindestens 10 Gew.-%, besonders bevorzugt mindestens 12 Gew.-%, und ganz besonders bevorzugt von mindestens 15 Gew.-%, bezogen auf das Gesamtgewicht der das Peptid-Antidiabetikum enthaltenden Schicht, in der wirkstoffhaltigen Schicht enthalten. Das Peptid-Antidiabetikum ist vorzugsweise in einer Menge von nicht mehr als 20 Gew.-%, besonders bevorzugt in einer Menge von nicht mehr als 16 Gew.-% in der wirkstoffhaltigen Schicht enthalten, bezogen auf das Gesamtgewicht der das Peptid-Antidiabetikum enthaltenden Schicht. Gemäß einer zusätzlichen und/oder alternativen Ausführungsform ist das mindestens eine Peptid-Antidiabetikum in einer Menge von etwa 20 Gew.-%, bezogen auf das Gesamtgewicht der das Peptid-Antidiabetikum enthaltenden Schicht, in der wirkstoffhaltigen Schicht enthalten.

Die mindestens eine wirkstoffhaltige Schicht umfasst eine Mischung von mindestens zwei Hydroxypropylmethylcellulosen, einer ersten Hydroxypropylmethylcellulose und einer zweiten Hydroxypropylmethylcellulose, in der das mindestens eine Peptid-Antidiabetikum enthalten ist. Gemäß einer zusätzlichen und/oder alternativen Ausführungsform umfasst die mindestens eine wirkstoffhaltige Schicht drei Hydroxypropylmethylcellulose, eine erste Hydroxypropylmethylcellulose, eine zweite Hydroxypropylmethylcellulose, und eine dritte Hydroxypropylmethylcellulose. Die unterschiedlichen Hydroxypropylmethylcellulosen unterscheiden sich in ihrer Viskosität voneinander.

Gemäß einer zusätzlichen und /oder alternativen Ausführungsform ist die erste Hydroxypropylmethylcellulose eine Hydroxypropylmethylcellulose mit hoher Viskosität. Unter einer Hydroxypropylmethylcellulose mit hoher Viskosität wird eine Hydroxypropylmethylcellulose mit einer Brookfield-Viskosität im Bereich von etwa 3.000 mPa·s bis etwa 5.600 mPa·s verstanden, gemessen mit einer 2 %-igen Lösung in Wasser bei einer Temperatur von 20 °C in einem Brookfield LV Viskosimeter. Derartige Hydroxypropylmethylcellulosen werden auch als HPMC 4000 bezeichnet.

Gemäß einer zusätzlichen und /oder alternativen Ausführungsform ist die zweite Hydroxypropylmethylcellulose eine Hydroxypropylmethylcellulose mit mittlerer Viskosität. Unter einer Hydroxypropylmethylcellulose mit mittlerer Viskosität wird eine Hydroxypropylmethylcellulose mit einer Brookfield-Viskosität im Bereich von etwa 75 mPa·s bis etwa 140 mPa·s verstanden, gemessen mit einer 2 %-igen Lösung in Wasser bei einer Temperatur von 20 °C in einem Brookfield LV Viskosimeter. Derartige Hydroxypropylmethylcellulosen werden auch als HPMC 100 bezeichnet.

Gemäß einer zusätzlichen und /oder alternativen Ausführungsform ist die dritte Hydroxypropylmethylcellulose eine Hydroxypropylmethylcellulose mit niedriger Viskosität. Unter einer HPMC mit niedriger Viskosität wird eine Hydroxypropylmethylcellulose mit einer Brookfield-Viskosität im Bereich von etwa 3 mPa·s verstanden, gemessen mit einer 2 %-igen Lösung in Wasser bei einer Temperatur von 20 °C in einem Brookfield LV Viskosimeter. Derartige Hydroxypropylmethylcellulosen werden auch als HPMC 3 bezeichnet.

Gemäß einer zusätzlichen und/oder alternativen Ausführungsform liegen die unterschiedlichen Hydroxypropylmethylcellulosen in einem Verhältnis von 1 : 6 bis 10 : 0 bis 6 (erste HPMC : zweiter HPMC : dritter HPMC). In einer besonderen Ausführungsform beträgt das Verhältnis von erster HPMC zu zweiter HPMC 1 : 9. Gemäß anderen Ausführungsformen beträgt das Verhältnis von erster HPMC zu zweiter HPMC zu dritter HPMC von 1 : 6,46 bis 6,52 : 4,07 bis 5,18. Gemäß einer anderen Ausführungsform beträgt das Verhältnis von erster HPMC zu zweiter HPMC zu dritter HPMC etwa 1 : 7,31 : 4,5.

Die Kombination der verschiedenen Hydroxypropylmethylcellulosen mit unterschiedlicher Viskosität, insbesondere in den vorgenannten Verhältnissen führt zu einem flexiblen Film, der an der Mundschleimhaut haftet und kein unangenehmes Fremdkörpergefühl verursacht.

Gemäß einer zusätzlichen und/oder alternativen Ausführungsform ist das Gemisch verschiedener Hydroxypropylmethylcellulosen, jeweils bezogen auf das Gesamtgewicht der wirkstoffhaltigen Schicht, in einer Menge von mindestens 49 Gew.-%, vorzugsweise mindestens 55 Gew.-%, besonders bevorzugt von mindestens 58 Gew.-% und ganz besonders bevorzugt in einer Menge von mindestens 60 Gew.-% in der mindestens einen wirkstoffhaltigen Schicht enthalten. Das Gemisch verschiedener Hydroxypropylmethylcellulosen ist in einer Menge von nicht mehr als 74 Gew.-%, vorzugsweise in einer Menge von nicht mehr als 65 Gew.-%, besonders bevorzugt in einer Menge von nicht mehr als 62 Gew.-%, und ganz besonders bevorzugt in einer Menge von nicht mehr als 61 Gew.-% in der mindestens einen wirkstoffhaltigen Schicht enthalten. Gemäß einer zusätzlichen und/oder alternativen Ausführungsform ist das Gemisch verschiedener Hydroxypropylmethylcellulosen in einer Menge von etwa 51 Gew.-% in der mindestens einen wirkstoffhaltigen Schicht enthalten.

Gemäß einer zusätzlichen und/oder alternativen Ausführungsform enthält die mindestens eine wirkstoffhaltige Schicht mindestens einen Weichmacher. Der mindestens eine Weichmacher erhöht die Flexibilität der filmförmigen Darreichungsform, so dass sich die Darreichungsform leichter an die Oberfläche der Mundschleimhaut anschmiegen kann und dabei kein unangenehmes Fremdkörpergefühl hervorruft.

Der mindestens eine Weichmacher kann aus der Gruppe von Weichmachern ausgewählt sein, die mittelkettige Triglyceride, Glycerol und Citronensäureester, zum Beispiel Triethylcitrat, umfasst. Mittelkettige Triglyceride sind Triglyceride, die mittelkettige Fettsäurereste enthalten. Mittelkettige Fettsäurereste weisen eine Kohlenstoffkette mit einer Länge von 6 bis 12 Kohlenstoffatomen auf.

Gemäß einer zusätzlichen und/oder alternativen Ausführungsform ist der mindestens eine Weichmacher oder die Weichmacher in einer Menge von mindestens 4 Gew.-%, vorzugsweise von mindestens 5 Gew.-%, besonders bevorzugt von mindestens 6 Gew.-% und ganz besonders bevorzugt von mindestens 9 Gew.-% in der mindestens einen wirkstoffhaltigen Schicht enthalten. Der mindestens eine Weichmacher oder die Weichmacher ist/sind in der mindestens einen wirkstoffhaltigen Schicht vorzugsweise in einer Menge von nicht mehr als 20 Gew.-%, besonders bevorzugt in einer Menge von nicht mehr als 16,5 Gew.-%, und ganz besonders bevorzugt in einer Menge von nicht mehr als 11 Gew.-% enthalten. Gemäß einer zusätzlichen und/oder alternativen Ausführungsform ist der mindestens eine Weichmacher oder die Weichmacher in einer Menge von etwa 16 Gew.-% in der mindestens einen wirkstoffhaltigen Schicht enthalten.

Gemäß einer zusätzlichen und/oder alternativen Ausführungsform umfasst die mindestens eine wirkstoffhaltige Schicht mindestens einen Enhancer. Der mindestens eine Enhancer verbessert die Permeabilität des Wirkstoffs durch die Mundschleimhaut.

Der mindestens eine Enhancer kann aus der Gruppe ausgewählt sein, die Betacyclodextrin, Polyoxyethylen-23-laurylether, Isopropylmyristat, Sojalecithin, Trehalose und Glycerol, Linolsäure, Ölsäure, Octyldodecanol, Polyethylenglycol und Gallensalze umfasst.

Der mindestens eine Enhancer oder die Summe der Enhancer kann in der wirkstoffhaltigen Schicht in einer Menge von mindestens 1 Gew.-%, vorzugsweise mindestens 1,5 Gew.-%, besonders bevorzugt mindestens 4 Gew.-% und ganz besonders bevorzugt von mindestens 5 Gew.-% enthalten sein. Der mindestens eine Enhancer bzw. die Enhancer sind in der wirkstoffhaltigen Schicht in einer Menge von nicht mehr als 22 Gew.-% enthalten, vorzugsweisen in einer Menge von nicht mehr als 18 Gew.-%, besonders bevorzugt in einer Menge von nicht mehr als 16 Gew.-%. Gemäß einer zusätzlichen und/oder alternativen Ausführungsform ist der mindestens eine Enhancer oder die Summe der Enhancer in einer Menge von etwa 16 Gew.-% in der mindestens einen wirkstoffhaltigen Schicht enthalten. Gemäß einer zusätzlichen und/oder alternativen Ausführungsform umfasst die mindestens eine wirkstoffhaltige Schicht mindestens einen Stabilisator. Der mindestens eine Stabilisator verbessert die Stabilität des Wirkstoffs in der wirkstoffhaltigen Schicht, insbesondere während der Lagerung der filmförmigen Zubereitung und erhöht somit die Haltbarkeitsdauer.

Der mindestens eine Stabilisator kann aus der Gruppe ausgewählt sein, die Betacyclodextrin, Dinatriumedetat, Poloxamer 188 und Trehalose umfasst.

Der mindestens eine Stabilisator bzw. die Summe der Stabilisatoren kann in der wirkstoffhaltigen Schicht in einer Menge von mindestens 0,1 Gew.-%, vorzugsweise mindestens 1,4 Gew.-%, besonders bevorzugt mindestens 3,5 Gew.-% enthalten sein. Der mindestens eine Stabilisator bzw. die Summe der Stabilisatoren ist in einer Menge von nicht mehr als 23 Gew.-%, vorzugsweise von nicht mehr als 16 Gew.-% enthalten, besonders bevorzugt nicht mehr als 11 Gew.-%, ganz besonders bevorzugt nicht mehr als 8 Gew.-%. Gemäß einer zusätzlichen und/oder alternativen Ausführungsform ist der Stabilisator oder die Summe der Stabilisatoren in einer Menge von 2,5 Gew.-% in der mindestens einen wirkstoffhaltigen Schicht enthalten.

Gemäß einer zusätzlichen und/oder alternativen Ausführungsform umfasst die mindestens eine wirkstoffhaltige Schicht mindestens ein Konservierungsmittel. Das Konservierungsmittel verbessert die Haltbarkeitsdauer der filmförmigen Zubereitung, insbesondere während der Lagerung.

Das mindestens eine Konservierungsmittel kann aus der Gruppe ausgewählt sein, die Methyl-4-benzoat umfasst.

Das mindestens eine Konservierungsmittel ist in einer Menge von etwa 0,1 Gew.-% enthalten.

Gemäß einer zusätzlichen und/oder alternativen Ausführungsform enthält die mindestens eine wirkstoffhaltige Schicht mindestens einen Farbstoff.

Der Farbstoff kann aus der Gruppe ausgewählt sein, die Titandioxid und Eisenoxid umfasst.

Der Farbstoff ist vorzugsweise in einer Menge von mindestens 0,1, vorzugsweise mindestens 0,5 Gew.-% besonders bevorzugt von mindestens 1 Gew.-%, und vorzugsweise in einer Menge von nicht mehr als 2 Gew.-% enthalten. Gemäß einer zusätzlichen und/oder alternativen Ausführungsform ist der Farbstoff in einer Menge von etwa 0,5 Gew.-% in der mindestens einen wirkstoffhaltigen Schicht enthalten.

Gemäß zusätzlichen und/oder alternativen Ausführungsformen enthält die mindestens eine wirkstoffhaltige Schicht mindestens einen weiteren pharmazeutisch annehmbaren Hilfsstoff. Der mindestens eine weitere pharmazeutisch annehmbare Hilfsstoff kann eine Puffersubstanz sein, beispielsweise einen Phosphatpuffer oder einen Citratpuffer, Na₂HPO₄, NaH₂PO₄, Trinatriumcitrat, ein Säuerungsmittel, beispielsweise Salzsäure oder Citronensäure, und/oder ein Säureregulator, beispielsweise Natriumhydroxid.

Der Phosphatpuffer kann in einer Menge von bis zu etwa 4,5 Gew.-% in der mindestens einen wirkstoffhaltigen Schicht enthalten sein. Gemäß einer zusätzlichen und/oder alternativen Ausführungsform kann Na₂HPO₄ in einer Menge von bis zu etwa 1,0 Gew.-% in der mindestens einen wirkstoffhaltigen Schicht enthalten sein. Gemäß einer zusätzlichen und/oder alternativen Ausführungsform kann NaH₂PO₄ in einer Menge von bis zu etwa 3,5 Gew.-% in der mindestens einen wirkstoffhaltigen Schicht enthalten sein.

Der Citratpuffer kann in einer Menge von bis zu 11,7 Gew.-% in der mindestens einen wirkstoffhaltigen Schicht enthalten sein. Gemäß einer zusätzlichen und/oder alternativen Ausführungsform kann Trinatriumcitrat in einer Menge von bis zu etwa 8,7 Gew.-% in der mindestens einen wirkstoffhaltigen Schicht enthalten sein. Gemäß einer zusätzlichen und/oder alternativen Ausführungsform kann Citronensäure in einer Menge von bis zu etwa 3,0 Gew.-% in der mindestens einen wirkstoffhaltigen Schicht enthalten sein. Gemäß einer zusätzlichen und/oder alternativen Ausführungsform ist Trinatriumcitrat in einer Menge von etwa 5,8 Gew.-% und/oder Citronensäure in einer Menge von etwa 3,0 Gew.-% in der mindestens einen wirkstoffhaltigen Schicht enthalten.

Der Säureregulator kann in einer Menge von bis zu etwa 1,0 Gew.-% in der mindestens einen wirkstoffhaltigen Schicht enthalten sein.

Gemäß einer zusätzlichen und/oder alternativen Ausführungsform umfasst die mindestens eine wirkstoffhaltige Schicht die folgende Zusammensetzung:

| Komponente | Anteil (Gew.-%) |
|---|---|
| Polymer | 50 - 74 |
| Weichmacher | 9 - 20 |
| Wirkstoff | 5,9 - 20 |
| Enhancer | 0 - 20 |
| Stabilisator | 1,4 - 22,9 |
| Konservierungsmittel | 0,1 - 0,2 |
| Farbstoff | 0 - 2,0 |
| Puffer | 0 - 4,5 |
| Säureregulator | 0 - 1 |
| Säuerungsmittel | 0 - 3 |
| Summe: | 100 |

Gemäß einer zusätzlichen und/oder alternativen Ausführungsform umfasst die filmförmige Darreichungsform eine Deckschicht. Die Deckschicht ist auf einer der beiden Flächen der wirkstoffhaltigen Schicht bzw. des Laminats aus mindestens zwei wirkstoffhaltigen Schichten angeordnet und bei korrekter Applikation der Darreichungsform auf einer Mundschleimhaut dem Mundraum zugewandt.

Die Deckschicht der filmförmigen Darreichungsform ist im Wesentlichen wirkstofffrei und sorgt für eine gerichtete Abgabe des mindestens einen Peptid-Antidiabetikums an die Mundschleimhaut, weil sie eine Freisetzung des in der Darreichungsform enthaltenen Wirkstoffs in den Mundraum verhindert. Für diesen Zweck ist die Deckschicht in Speichel unlöslich oder löst bzw. zerfällt in Speichel wesentlich langsamer als die wirkstoffhaltige Schicht. Vorzugsweise ist die Deckschicht für den in der wirkstoffhaltigen Schicht oder den wirkstoffhaltigen Schichten enthaltenen Wirkstoff im Wesentlichen undurchlässig.

Gemäß einer zusätzlichen und/oder alternativen Ausführungsform umfasst die mindestens eine Deckschicht einen Gehalt an einem Polymer, vorzugsweise einen Gehalt an Ethylcellulose, weil Polymerfilme aus Ethylcellulose sehr flexibel sind. Der Gehalt an Polymer, insbesondere an Ethylcellulose, in der Deckschicht beträgt mindestens 50 Gew.-%, vorzugsweise mindestens 55 Gew.-%, besonders bevorzugt mindestens 60 Gew.-%, und ganz besonders bevorzugt mindestens 65 Gew.-%, bezogen auf das Gesamtgewicht der Deckschicht. Der Gehalt an Polymer, insbesondere an Ethylcellulose, in der Deckschicht beträgt maximal 99 Gew.-%, vorzugsweise maximal 95 Gew.-%, besonders bevorzugt maximal 94 Gew.-%, und ganz besonders bevorzugt maximal 93,5 Gew.-%, bezogen auf das Gesamtgewicht der Deckschicht.

Gemäß einer besonderen Ausführungsform handelt es sich bei der Ethylcellulose um eine Ethylcellulose mit einer Viskosität im Bereich von 90 bis 110 mPa·s (cP), gemessen mit einem Ubbelohde Viskosimeter bei 25 °C für eine 5 %-ige Lösung in einem Lösungsmittelgemisch aus 80 Vol.-% Toluol und 20 Vol.-% Ethanol. Derartige Ethylcellulosen werden auch als Ethylcellulose 100 bezeichnet.

Gemäß einer zusätzlichen und/oder alternativen Ausführungsform umfasst die Deckschicht mindestens einen Weichmacher. Der Gehalt an Weichmacher in der Deckschicht beträgt vorzugsweise mindestens 5 Gew.-%, besonders bevorzugt mindestens 10 Gew.-%, und ganz besonders bevorzugt mindestens 15 Gew.-%, bezogen auf das Gesamtgewicht der Deckschicht. Der Gehalt an Weichmacher in der Deckschicht beträgt vorzugsweise nicht mehr als 34 Gew.-%, besonders bevorzugt nicht mehr als 30 Gew.-%, und ganz besonders bevorzugt nicht mehr als 20 Gew.-%, bezogen auf das Gesamtgewicht der Deckschicht.

Der Weichmacher in der Deckschicht kann aus der Gruppe ausgewählt sein, die mittelkettige Triglceride, Glycerol und Citronensäureester wie beispielsweise Triethylcitrat umfasst. Mittelkettige Triglyceride sind Triglyceride, die mittelkettige Fettsäuren enthalten. Zu den mittelkettigen Fettsäuren zählen die Fettsäuren mit Kohlenstoffketten von 6 bis 12 C-Atomen, beispielsweise die Capronsäure- (C 6:0), die Caprylsäure- (C 8:0), die Caprinsäure (C 10:0) und die Laurinsäure (C 12:0).

Gemäß einer zusätzlichen und/oder alternativen Ausführungsform weist die Deckschicht einen Farbstoff auf. Der Gehalt an Farbstoff in der Deckschicht beträgt zwischen etwa 0,5 Gew.-% und etwa 2 Gew.-%, vorzugsweise etwa 1 Gew.-% oder etwa 1,5 Gew.-%, bezogen auf das Gesamtgewicht der Deckschicht.

Der Farbstoff kann aus der Gruppe ausgewählt sein, die aus Titandioxid und Eisenoxid besteht.

Gemäß einer zusätzlichen und/oder alternativen Ausführungsform unterscheiden sich die Deckschicht und die mindestens eine wirkstoffhaltige Schicht farblich voneinander. Dadurch kann der Patient die wirkstoffhaltige Schicht von der Deckschicht unterscheiden und die filmförmige Darreichungsform in richtiger Orientierung applizieren, so dass die Deckschicht nicht an der Mundschleimhaut anliegt, sondern dem Mundraum zugewandt ist.

Gemäß dem zweiten Aspekt betrifft die Erfindung ein Verfahren zur Herstellung der vorstehend beschriebenen filmförmigen Darreichungsformen.

Gemäß einer Ausführungsform des Verfahrens werden die filmförmigen Darreichungsformen hergestellt, indem die Komponenten für die wirkstoffhaltige(n) Schicht(en) in einem Lösungsmittel gelöst oder suspendiert werden. Die resultierende viskose Masse wird mit einem geeigneten Filmziehgerät, beispielsweise einer Rakel, auf einen flächigen Träger in definierter Dicke aufgebracht. Anschließend wird der so erhaltene Wirkstoffstrich getrocknet.

Das Verfahren umfasst die Schritte:
Lösen und/oder Suspendieren der Komponenten für die wirkstoffhaltige Schicht in einem Lösungsmittel,
Aufbringen der Lösung oder Suspension auf einen Träger in definierter Dicke; und
anschließendes Trocknen.

Gemäß einer Ausführungsform ist das Lösungsmittel für die wirkstoffhaltige Schicht aus der Gruppe ausgewählt, die Ethanol, Wasser und Gemische aus Wasser und Ethanol, insbesondere ein Gemisch aus etwa 96 Vol.-% Ethanol und etwa 4 Vol.-% Wasser (96 %-iger Ethanol), umfasst. Gemäß einer zusätzlichen Ausführungsform weist das Gemisch aus Wasser und Ethanol ein Volumenverhältnis von 80 % zu 20 % (Wasser zu Ethanol) auf. Überraschenderweise konnte durch Verwendung der genannten Gemische aus Wasser und Ethanol eine bessere Wirkstoffpermeation aus der resultierenden filmförmigen Darreichungsform erzielt werden, bezogen auf Insulin, als bei Verwendung von Wasser als Lösungsmittel.

Gemäß einer weiteren Ausführungsform ist der Träger ein flächiger Träger, beispielsweise eine Kunststoff-Folie, vorzugsweise eine Polyethylenterephthalat-Folie, eine vorgefertigte wirkstoffhaltige Schicht, eine vorgefertigte Deckschicht, ein vorgefertigtes Laminat umfassend mindestens eine wirkstoffhaltige Schicht, oder ein vorgefertigtes Laminat umfassend eine Deckschicht und mindestens eine wirkstoffhaltige Schicht.

Gemäß einer Ausführungsform wird bei einer Temperatur von nicht mehr als 80 °C, vorzugsweise von nicht mehr als 50 °C, besonders bevorzugt bei einer Temperatur von etwa 50 °C getrocknet. Durch die Trocknung wird das Lösungsmittel aus der Schicht entfernt. Durch die relativ niedrige Trocknungstemperatur wird sichergestellt, dass das Peptid-Antidiabetikum während der Trocknung nicht zerstört wird.

Gemäß einer zusätzlichen und/oder alternativen Ausführungsform wird mindestens eine weitere wirkstoffhaltige Schicht auf einen bereits getrockneten Wirkstoffstrich aufgebracht, indem die Komponenten für die weitere wirkstoffhaltige Schicht in einem Lösungsmittel gelöst und/oder suspendiert werden,
die Lösung oder Suspension auf den getrockneten Wirkstoffstrich in definierter Dicke aufgebracht wird, und
der so erhaltene weitere Wirkstoffstrich getrocknet wird. Insofern repräsentiert der getrocknete Wirkstoffstrich, auf den die Lösung oder Suspension für den weiteren Wirkstoffstrich aufgebracht wird, den flächigen Träger.

Vorzugsweise wird für die Herstellung der weiteren wirkstoffhaltigen Schicht(en) das gleiche Lösungsmittel verwendet wie für die Herstellung der ersten wirkstoffhaltigen Schicht.

Vorzugsweise erfolgt die Trocknung der weiteren wirkstoffhaltigen Schicht(en) bei einer Temperatur von nicht mehr als 80 °C, vorzugsweise von nicht mehr als 50 °C, besonders bevorzugt bei einer Temperatur von etwa 50 °C.

Gemäß einer weiteren und/oder alternativen Ausführungsform für die Herstellung von filmförmigen Darreichungsformen gemäß der vorliegenden Erfindung, die eine Deckschicht aufweisen, werden die Komponenten für die Deckschicht in einem Lösungsmittel gelöst oder suspendiert. Die so erhaltene viskose Masse wird mit einem geeigneten Filmziehgerät, beispielsweise einer Rakel, auf den letzten getrockneten Wirkstoffstrich als flächigen Träger aufgebracht und ebenfalls getrocknet.

Bei dieser Ausführungsform umfasst das Verfahren zur Herstellung der filmförmigen Darreichungsform,
das Lösen und/oder Suspendieren der Komponenten für die Deckschicht in einem Lösungsmittel,
das Aufbringen der Lösung oder Suspension für die Deckschicht auf den letzten getrockneten Wirkstoffstrich, und
das Trocknen der Deckschicht.
Gemäß einer Ausführungsform ist das Lösungsmittel für die Deckschicht aus der Gruppe ausgewählt, die Wasser und Gemische aus Wasser und Ethanol umfasst. Gemäß einer zusätzlichen Ausführungsform weist das Gemisch aus Wasser und Ethanol ein Volumenverhältnis von 80 % zu 20 % (Wasser zu Ethanol) auf. Gemäß einer alternativen Ausführungsform ist das Lösungsmittel für die Deckschicht Ethanol (96 %-ig).

Gemäß einer Ausführungsform wird die Deckschicht bei einer Temperatur von nicht mehr als 80 °C, vorzugsweise von nicht mehr als 50 °C, besonders bevorzugt bei einer Temperatur von etwa 50 °C getrocknet. Durch die Trocknung wird das Lösungsmittel aus der Schicht entfernt. Durch die relativ niedrige Trocknungstemperatur wird sichergestellt, dass das Peptid-Antidiabetikum während der Trocknung nicht zerstört wird.

Gemäß einer alternativen Ausführungsform für die Herstellung filmförmiger Darreichungsformen gemäß der vorliegenden Erfindung, die eine Deckschicht aufweisen, wird zunächst die Deckschicht hergestellt, indem eine Lösung oder Suspension mit den Komponenten für die Deckschicht auf einem flächigen Träger, vorzugsweise einer Kunststoff-Folie, besonders bevorzugt einer Polyethylenterepthalat-Folie, in definierter Dicke aufgetragen und der so erhaltene Materialstrich getrocknet wird. Anschließend wird mindestens eine wirkstoffhaltige Schicht auf der vorgefertigten Deckschicht als Träger für die wirkstoffhaltige Schicht in definierter Dicke aufgebracht und getrocknet.

Für diese Herstellungsvariante werden Komponenten für die Deckschicht in einem Lösungsmittel, vorzugsweise in Wasser, Ethanol oder einem Gemisch aus Wasser und Ethanol, insbesondere einem Gemisch aus 80 Vol.-% Wasser und 20 Vol.-% Ethanol, oder Ethanol (96-%ig), gelöst oder suspendiert. Die so erhaltene viskose Masse wird mit einem geeigneten Gerät, beispielsweise einer Rakel, auf einen Träger, vorzugsweise eine Kunststoff-Folie, beispielsweise einer Polyethylenterephthalat-Folie, in definierter Dicke aufgebracht. Der so erhaltene Materialstrich wird getrocknet, vorzugsweise wie vorstehend beschrieben. Da bei dieser Trocknung kein Wirkstoff durch die erhöhte Trocknungstemperatur beeinträchtigt werden kann, ist es auch möglich, den Materialstrich für die Deckschicht bei höheren Temperaturen zu trocknen. Dadurch kann die Trocknungszeit verkürzt und das Herstellungsverfahren insgesamt beschleunigt werden.

Nach dem Trocknen wird eine wirkstoffhaltige Schicht auf die vorgefertigte Deckschicht aufgebracht. Dazu werden die Komponenten für die wirkstoffhaltige Schicht in einem Lösungsmittel gelöst oder suspendiert, vorzugweise in Wasser, Ethanol oder einem Gemisch aus Wasser und Ethanol, insbesondere einem Gemisch aus 80 Vol.-% Wasser und 20 Vol.-% Ethanol, oder Ethanol (96-%ig). Die resultierende viskose wirkstoffhaltige Masse wird mit einem geeigneten Filmziehgerät, beispielsweise einer Rakel, in definierter Dicke auf die freie Fläche der vorgefertigten Deckschicht aufgebracht.

Anschließend wird der so erhaltene Wirkstoffstrich getrocknet. Gemäß einer Ausführungsform wird bei einer Temperatur von nicht mehr als 80 °C, vorzugsweise von nicht mehr als 50 °C, besonders bevorzugt bei einer Temperatur von etwa 50 °C getrocknet.

Das Verfahren gemäß dieser Ausführungsform umfasst somit die Schritte: Lösen und/oder Suspendieren der Komponenten für die Deckschicht in einem Lösungsmittel;
Aufbringen der Lösung auf einen flächigen Träger in definierter Dicke; Trocknen der Deckschicht;
Lösen oder Suspendieren der Komponenten für die wirkstoffhaltige Schicht in einem Lösungsmittel;
Aufbringen der wirkstoffhaltigen Lösung auf die vorgefertigte Deckschicht; und
Trocknen des so erhaltenen Wirkstoffstrichs.

Das Herstellen einer filmförmigen Darreichungsform zur Verabreichung eines Peptid-Antidiabetikums durch Vorfertigen der Deckschicht und nachfolgendes Aufbringen der mindestens einen wirkstoffhaltigen Schicht ist für den Wirkstoff schonender, weil das Peptid-Antidiabetikum nur einer Trocknungsphase unterworfen werden muss, und eine weitere Beeinträchtigung des Wirkstoffs durch die Trocknung der Deckschicht vermieden werden kann. Dieses für den Wirkstoff schonendere Herstellungsverfahren ist vorteilhaft, weil die Wirksamkeit des Wirkstoffs in der Darreichungsform möglichst wenig beeinträchtigt wird.

In einer weiteren Ausführungsform kann mindestens eine weitere wirkstoffhaltige Schicht auf den bereits getrockneten und auf die vorgefertigte Deckschicht aufgebrachten Wirkstoffstrich aufgebracht werden, vorzugsweise wie vorstehend für den Wirkstoffstrich und/oder den mindestens einen weiteren Wirkstoffstrich beschrieben.

Gemäß einer weiteren Ausführungsform des Herstellungsverfahrens werden die Darreichungsformen aus dem wirkstoffhaltigen Film oder dem resultierenden wirkstoffhaltigen Laminat, umfassend mehrere wirkstoffhaltige Schichten oder mindestens eine wirkstoffhaltige Schicht und die Deckschicht, vereinzelt, beispielsweise durch Schneiden oder Stanzen. Die vereinzelten Darreichungsformen werden anschließend verpackt, vorzugsweise einzeln verpackt.

Gemäß einer anderen Ausführungsform des Herstellungsverfahrens wird der wirkstoffhaltige Film oder das resultierende Laminat, umfassend mehrere wirkstoffhaltige Schichten oder mindestens eine wirkstoffhaltige Schicht und die Deckschicht, in einen oder mehrere Streifen vorbestimmter Breite und Länge geschnitten. Die so erhaltenen wirkstoffhaltigen Streifen werden als Rollenware oder Leporello-gefaltet verpackt. Eine derartige Konfektionierung bietet den Vorteil, dass sich ein Patient je nach individuellem Bedarf einen Abschnitt vom wirkstoffhaltigen Streifen mit der erforderlichen Wirkstoffmenge abtrennen kann, so dass er sich mit der auf diese Weise vereinzelten Darreichungsform eine auf seinen individuellen Bedarf abgemessene Wirkstoffmenge verabreichen kann.

Gemäß einer zusätzlichen Ausführungsform weist der Streifen vorbestimmter Länge Kennzeichnungen auf, die sich auf eine vorbestimmte Menge an Wirkstoff in dem wirkstoffhaltigen Streifen beziehen. Vorzugsweise sind die Kennzeichnungen auf dem wirkstoffhaltigen Streifen in gleichem Abstand zueinander angeordnet. Bei den Kennzeichnungen kann es sich um Linien, Muster, Perforationen, Oberflächenstrukturen, Einkerbungen, Schwächungen oder dergleichen handeln. Derartige Kennzeichnungen erleichtern dem Anwender das präzise Dosieren des Wirkstoffs durch das Abtrennen eines Abschnitts, der die gewünschte Wirkstoffmenge enthält.

Gemäß einer zusätzlichen und/oder Alternativen Ausführungsform wird der wirkstoffhaltige Streifen in einen Spender verpackt. Die Verpackung des wirkstoffhaltigen Streifens in einen Spender ist vorteilhaft, weil der wirkstoffhaltige Streifen in dieser Ausführungsform sofort zur Nutzung durch einen Patienten zur Verfügung steht, der wirkstoffhaltige Streifen selbst keiner Handhabung (Auspacken, Befüllen eines Spenders, etc.) durch den Patienten bedarf und dennoch in einer Form vorliegt, die eine besonders benutzerfreundliche Nutzung bereitstellt, beispielsweise weil der Spender eine besonders genaue Dosierung und/oder ein besonders leichtes Abtrennen des gewünschten Abschnitts ermöglicht.

Die wirkstoffhaltigen Streifen können alternativ auch einzeln verpackt werden, nicht in einem Spender. In dieser Ausgestaltung können die einzeln verpackten wirkstoffhaltigen Streifen als Nachfüllpackung für einen entsprechenden Spender genutzt werden.

Gemäß dem dritten Aspekt betrifft die Erfindung die Verwendung der vorstehend beschriebenen filmförmigen Darreichungsformen zur transmukosalen Verabreichung eines Peptid-Antidiabetikums, insbesondere zur Verabreichung mindestens über eine Mundschleimhaut. Die Erfindung erstreckt sich somit auch auf ein Verfahren zur Verabreichung mindestens eines Peptid-Antidiabetikums über die Mundschleimhaut, ebenso wie auf Verfahren zur Behandlung von Patienten, die der Verabreichung eines Peptid-Antidiabetikums bedürfen.

Gemäß einer bevorzugten Ausführungsform der Verwendung der filmförmigen Darreichungsform beziehungsweise der Verfahren zur Verabreichung mindestens eines Peptid-Antidiabetikums oder des Verfahrens zur Behandlung eine Patienten, der der Verabreichung eines Peptid-Antidiabetikums bedarf mittels der filmförmigen Darreichungsform erfolgt die Verabreichung der filmförmigen Darreichungsform auf der Schleimhaut des Zahnfleisches (gingivale Applikation). Dieser Applikationsort für die erfindungsgemäßen Darreichungsformen hat sich überraschenderweise als besonders günstig herausgestellt, insbesondere im Hinblick auf die Permeation des Peptid-Antidiabetikums über die Schleimhaut.

Gemäß einer Ausführungsform enthält die filmförmige Darreichungsform Insulin oder ein Insulinanalogon als Wirkstoff und wird zur Behandlung der Zuckerkrankheit, des Diabetes mellitus Typ 1 oder des Diabetes mellitus Typ 2 verwendet.

Die transmukosale Verabreichung, insbesondere die orale Verabreichung und anschließende Absorption des Peptid-Antidiabetikums über die Mundschleimhaut ist für den Anwender besonders komfortabel. Der Wirkstoff diffundiert direkt durch die Schleimhaut in den Blutstrom, wodurch im Allgemeinen auch eine "first pass" Verstoffwechselung vermieden wird. Diese Methode der Verabreichung ist einfach auszuführen und damit auch für Patienten mit behindertem Sehvermögen und/oder eingeschränktem Koordinationsvermögen geeignet.

Die Erfindung wird nachfolgend anhand von Ausführungsbeispielen und mit Bezug auf die Figuren veranschaulicht. Dabei schränken weder die Ausführungsbeispiele noch die Figuren die beanspruchte Erfindung ein, sondern dienen lediglich der Erläuterung.

### Beispiel 1

Die Komponenten für die wirkstoffhaltige Schicht wurden in Wasser, versetzt mit 0,141 Gew.-% einer 37 %-igen Salzsäure, gelöst und mit einem Filmziehgerät auf eine Polyethylenterephthalat-Folie aufgebracht. Der Wirkstoffstrich wurde anschließend bei einer Temperatur von 80 °C getrocknet.

Anschließend wurde mit dem Filmziehgerät ein weiterer Wirkstoffstrich auf die vorstehend beschriebene wirkstoffhaltige Schicht aufgebracht. Das Laminat wurde anschließend bei einer Temperatur von 80 °C getrocknet. Aus dem resultierenden Film wurden Einzeldosen à 4,11 cm² ausgestanzt.

| Komponente | Anteil in Gew.-% |
|---|---|
| Hydroxypropylmethylcellulose, 100 USP | 54,000 |
| Hydroxypropylmethylcellulose, 4000 USP | 6,000 |
| Mittelkettige Triglyceride | 6,000 |
| Glycerol Ph.Eur./USP | 4,500 |
| Insulin | 5,925 |
| Na₂HPO₄ | 0,970 |
| Na_{Z}HPO₄, wasserfrei | 3,500 |
| Betacyclodextrin | 10,405 |
| Poloxamer 188 (Blockcopolymer aus Ethylenoxid und | 5,000 |
| Propylenoxid mit einer mittleren relativen | |
| Molekülmasse von 7680 bis 9510) | |
| Polyoxyethylen-23-laurylether | 1,500 |
| Dinatriumedetat | 0,100 |
| Methyl-4-hydroxybenzoat | 0,100 |
| Titandioxid | 2,000 |
| Summe: | 100,000 |

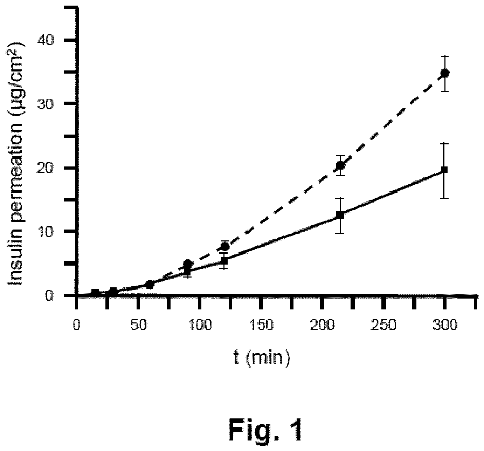

Die in einem in vitro-Permeationstest gemäß Beispiel 9 mit filmförmigen Darreichungsformen gemäß diesem Beispiel erzielbaren Permeationsraten sind in Fig. 1 in der mit - ■- gekennzeichneten Kurve dargestellt.

### Beispiel 2

Die Komponenten für die wirkstoffhaltige Schicht wurden in einem Lösungsmittelgemisch aus 20 Gew.-% Ethanol und 80 Gew.-% Wasser gelöst und mit einem Filmziehgerat auf eine Polyethylenterephthalat-Folie aufgebracht und anschließend bei einer Temperatur von 50 °C getrocknet.

Zusammensetzung der wirkstoffhaltigen Schicht

| Komponente | Anteil in Gew.-% |
|---|---|
| Hydroxypropylmethylcellulose, 100 USP | 34,424 |
| Hydroxypropylmethylcellulose, 4000 USP | 5,280 |
| Hydroxypropylmethylcellulose, 3 mPas | 22,000 |
| Triethylcitrat | 5,280 |
| Glycerol Ph.Eur./USP | 5,280 |
| Insulin | 12,000 |
| Na_{Z}HPO₄, wasserfrei | 2,640 |
| Betacyclodextrin | 4,998 |
| Poloxamer 188 (Blockcopolymer aus Ethylenoxid und | 5,500 |
| Propylenoxid mit einer mittleren relativen | |
| Molekülmasse von 7680 bis 9510) | |
| Polyoxyethylen-23-laurylether | 1,601 |
| Dinatriumedetat | 0,320 |
| Methyl-4-hydroxybenzoat | 0,114 |
| Salzsäure, 37 %-iq | 0,563 |
| Summe: | 100,000 |

Die Komponenten für die Deckschicht wurden in Ethanol (96 %-ig) gelöst und mit einem Filmziehgerat auf die vorstehend beschriebene wirkstoffhaltige Schicht aufgebracht. Das Laminat wurde anschließend bei einer Temperatur von 50 °C getrocknet.

Bei dem resultierenden zweischichtigen Film hatte die getrocknete Deckschicht ein Flächengewicht von 48,0 g/m² und die wirkstoffhaltige Schicht ein Flächengewicht von 114 g/m². Aus dem Film wurden Einzeldosen à 4,11 cm² ausgestanzt.

Zusammensetzung der Deckschicht:

| Komponente | Anteil in Gew.-% |
|---|---|
| Ethylcellulose 100 | 93,500 |
| Triethylcitrat | 5,000 |
| Eisenoxid, gelb | 1,500 |
| Summe | 100,000 |

Die in einem *in vitro*-Permeationstest gemäß Beispiel 9 mit filmförmigen Darreichungsformen gemäß diesem Beispiel erzielbaren Permeationsraten sind in Fig. 1 in der mit - • - gekennzeichneten Kurve dargestellt.

### Beispiel 3

Die Komponenten für die wirkstoffhaltige Schicht wurden in einem Lösungsmittelgemisch aus 20 Gew.-% Ethanol und 80 Gew.-% Wasser gelöst und mit einem Filmziehgerat auf eine Polyethylenterephthalat-Folie aufgebracht und anschließend bei einer Temperatur von 50 °C getrocknet.

Zusammensetzung der wirkstoffhaltigen Schicht

| Komponente | Anteil in Gew.-% |
|---|---|
| Hydroxypropylmethylcellulose, 100 USP | 34,050 |
| Hydroxypropylmethylcellulose, 4000 USP | 5,220 |
| Hydroxypropylmethylcellulose, 3 mPas | 21,240 |
| Triethylcitrat | 5,200 |
| Glycerol Ph.Eur./USP | 5,200 |
| Insulin aspart | 12,000 |
| Na_{Z}HPO₄, wasserfrei | 2,640 |
| Isopropylmyristat | 1,000 |
| Sojalecithin | 4,000 |
| Trehalose | 1,500 |
| Betacyclodextrin | 1,500 |
| Poloxamer 188 (Blockcopolymer aus Ethylenoxid und Propylenoxid mit einer mittleren relativen Molekülmasse von 7680 bis 9510) | 4,500 |
| Polyoxyethylen-23-laurylether | 1,500 |
| Dinatriumedetat | 0,350 |
| Methyl-4-hydroxybenzoat | 0,100 |
| Summe: | 100,000 |

Die Komponenten für die Deckschicht wurden in Ethanol (96 %-ig) gelöst und mit einem Filmziehgerät auf die vorstehend beschriebene wirkstoffhaltige Schicht aufgebracht. Das Laminat wurde anschließend bei einer Temperatur von 50 °C getrocknet.

Bei dem resultierenden zweischichtigen Film hatte die getrocknete Deckschicht ein Flächengewicht von 48,0 g/m² und die wirkstoffhaltige Schicht ein Flächengewicht von 114 g/m². Aus dem Film wurden Einzeldosen à 4,11 cm² ausgestanzt.

Zusammensetzung der Deckschicht:

| Komponente | Anteil in Gew.-% |
|---|---|
| Ethylcellulose 100 | 93,500 |
| Triethylcitrat | 5,000 |
| Eisenoxid, gelb | 1,500 |
| Summe | 100,000 |

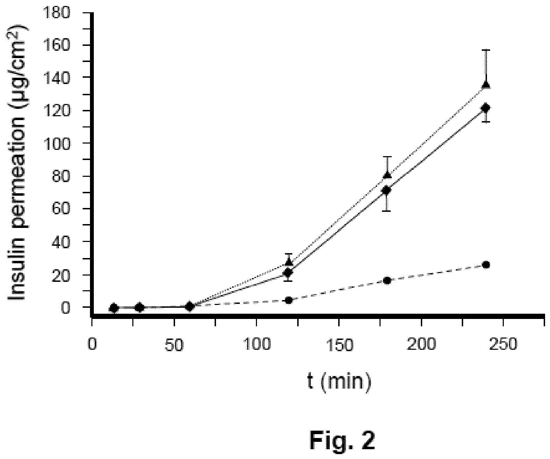

Die in einem in vitro-Permeationstest gemäß Beispiel 9 mit filmförmigen Darreichungsformen gemäß diesem Beispiel erzielbaren Permeationsraten sind in Fig. 2 in der mit -●- gekennzeichneten Kurve dargestellt.

### Beispiel 4

Zusammensetzung der wirkstoffhaltigen Schicht

| Komponente | Anteil in Gew.-% |
|---|---|
| Hydroxypropylmethylcellulose, 100 USP | 31,000 |
| Hydroxypropylmethylcellulose, 4000 USP | 4,800 |
| Hydroxypropylmethylcellulose, 3 mPas | 24,870 |
| Glycerol Ph.Eur./USP | 16,500 |
| Insulin aspart | 15,000 |
| Citronensäure | 3,000 |
| Natriumhydroxid | 0,880 |
| Trehalose | 1,500 |
| Betacyclodextrin | 1,500 |
| Dinatriumedetat | 0,350 |
| Methyl-4-hydroxybenzoat | 0,100 |
| Titandioxid | 0,500 |
| Summe: | 100,000 |

Die Komponenten für die wirkstoffhaltige Schicht wurden in einem Lösungsmittelgemisch aus 20 Gew.-% Ethanol und 80 Gew.-% Wasser gelöst und mit einem Filmziehgerat auf eine Polyethylenterephthalat-Folie aufgebracht und anschließend bei einer Temperatur von 50 °C getrocknet.

Zusammensetzung der Deckschicht:

| Komponente | Anteil in Gew.-% |
|---|---|
| Ethylcellulose 100 | 93,500 |
| Triethylcitrat | 5,000 |
| Eisenoxid, gelb | 1,500 |
| Summe | 100,000 |

Die Komponenten für die Deckschicht wurden in Ethanol (96 %-ig) gelöst und mit einem Filmziehgerat auf die vorstehend beschriebene wirkstoffhaltige Schicht aufgebracht. Das Laminat wurde anschließend bei einer Temperatur von 50 °C getrocknet.

Bei dem resultierenden zweischichtigen Film hatte die getrocknete Deckschicht ein Flächengewicht von 48,0 g/m² und die wirkstoffhaltige Schicht ein Flächengewicht von 114 g/m². Aus dem Film wurden Einzeldosen à 4,11 cm² ausgestanzt.

Die in einem in vitro-Permeationstest gemäß Beispiel 9 mit filmförmigen Darreichungsformen gemäß diesem Beispiel erzielbaren Permeationsraten sind in Fig. 2 in der mit -◆- gekennzeichneten Kurve dargestellt.

### Beispiel 5

Die Komponenten für die wirkstoffhaltige Schicht wurden in einem Lösungsmittelgemisch aus 20 Gew.-% Ethanol und 80 Gew.-% Wasser gelöst und mit einem Filmziehgerat auf eine Polyethylenterephthalat-Folie aufgebracht und anschließend bei einer Temperatur von 50 °C getrocknet.

Zusammensetzung der wirkstoffhaltigen Schicht

| Komponente | Anteil in Gew.-% |
|---|---|
| Hydroxypropylmethylcellulose, 100 USP | 31,000 |
| Hydroxypropylmethylcellulose, 4000 USP | 4,800 |
| Hydroxypropylmethylcellulose, 3 mPas | 20,070 |
| Glycerol Ph.Eur./USP | 16,500 |
| Insulin aspart | 15,000 |
| Citronensäure | 3,000 |
| Natriumhydroxid | 0,880 |
| Trinatriumcitrat | 5,800 |
| Trehalose | 1,000 |
| Betacyclodextrin | 1,000 |
| Dinatriumedetat | 0,350 |
| Methyl-4-hydroxybenzoat | 0,100 |
| Titandioxid | 0,500 |
| Summe: | 100,000 |

Die Komponenten für die Deckschicht wurden in Ethanol (96 %-ig) gelöst und mit einem Filmziehgerät auf die vorstehend beschriebene wirkstoffhaltige Schicht aufgebracht. Das Laminat wurde anschließend bei einer Temperatur von 50 °C getrocknet.

Zusammensetzung der Deckschicht:

| Komponente | Anteil in Gew.-% |
|---|---|
| Ethylcellulose 100 | 93,500 |
| Triethylcitrat | 5,000 |
| Eisenoxid, gelb | 1,500 |
| Summe | 100,000 |

Bei dem resultierenden zweischichtigen Film hatte die getrocknete Deckschicht ein Flächengewicht von 48,0 g/m² und die wirkstoffhaltige Schicht ein Flächengewicht von 114 g/m². Aus dem Film wurden Einzeldosen à 4,11 cm² ausgestanzt.

Die in einem in vitro-Permeationstest gemäß Beispiel 9 mit filmförmigen Darreichungsformen gemäß diesem Beispiel erzielbaren Permeationsraten sind in Fig. 2 in der mit -▲- gekennzeichneten Kurve dargestellt.

### Beispiel 6

Die Komponenten für die Deckschicht wurden in Ethanol (96 %-ig) gelöst, mit einem Filmziehgerat auf eine Polyethylenterephthalat-Folie aufgebracht und anschließend bei einer Temperatur von 50 °C getrocknet.

Zusammensetzung der Deckschicht:

| Komponente | Anteil in Gew.-% |
|---|---|
| Ethylcellulose 100 | 88,500 |
| Triethylcitrat | 5,000 |
| Hydroxypropylmethylcellulose 100 | 5,000 |
| Eisenoxid, gelb | 1,500 |
| Summe | 100,000 |

Die Komponenten für die wirkstoffhaltige Schicht wurden in einem Lösungsmittelgemisch aus 20 Gew.-% Ethanol und 80 Gew.-% Wasser gelöst und mit einem Filmziehgerat auf eine Polyethylenterephthalat-Folie aufgebracht und anschließend bei einer Temperatur von 50 °C getrocknet.

Zusammensetzung der wirkstoffhaltigen Schicht

| Komponente | Anteil in Gew.-% |
|---|---|
| Hydroxypropylmethylcellulose, 100 USP | 29,250 |
| Hydroxypropylmethylcellulose, 4000 USP | 4,000 |
| Hydroxypropylmethylcellulose, 3 mPas | 18,000 |
| Glycerol, wasserfrei Ph.Eur./USP | 16,000 |
| Insulin aspart | 20,000 |
| Citronensäure | 3,000 |
| Natriumhydroxid | 0,880 |
| Trinatriumcitrat | 5,800 |
| Trehalose | 1,000 |
| Betacyclodextrin | 1,000 |
| Dinatriumedetat | 0,470 |
| Methyl-4-hydroxybenzoat | 0,100 |
| Titandioxid | 0,500 |
| Summe: | 100,000 |

Bei dem resultierenden zweischichtigen Film hatte die getrocknete Deckschicht ein Flächengewicht von 48,0 g/m² und die wirkstoffhaltige Schicht ein Flächengewicht von 114 g/m². Aus dem Film wurden Einzeldosen à 4,11 cm² ausgestanzt.

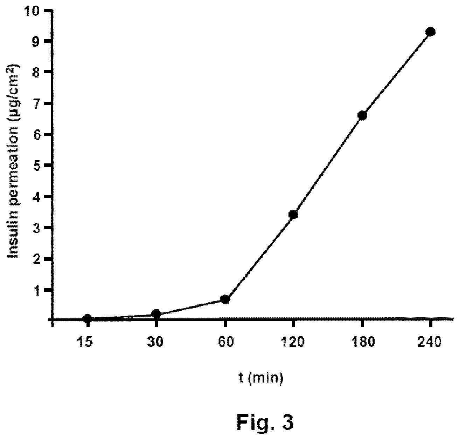

Die in einem in vitro-Permeationstest gemäß Beispiel 9 mit filmförmigen Darreichungsformen gemäß diesem Beispiel erzielbaren Permeationsraten sind in Fig. 3 graphisch dargestellt.

### Beispiel 7

Die Komponenten für die Deckschicht wurden in Ethanol (96 %-ig) gelöst und mit einem Filmziehgerat auf die vorstehend beschriebene wirkstoffhaltige Schicht aufgebracht. Das Laminat wurde anschließend bei einer Temperatur von 50 °C getrocknet.

Zusammensetzung der Deckschicht:

| Komponente | Anteil in Gew.-% |
|---|---|
| Ethylcellulose 100 | 93,500 |
| Triethylcitrat | 5,000 |
| Eisenoxid, gelb | 1,500 |
| Summe | 100,000 |

Die Komponenten für die wirkstoffhaltige Schicht wurden in einem Lösungsmittelgemisch aus 20 Gew.-% Ethanol und 80 Gew.-% Wasser gelöst und mit einem Filmziehgerat auf eine Polyethylenterephthalat-Folie aufgebracht und anschließend bei einer Temperatur von 50 °C getrocknet.

Zusammensetzung der wirkstoffhaltigen Schicht

| Komponente | Anteil in Gew.-% |
|---|---|
| Hydroxypropylmethylcellulose, 100 USP | 34,424 |
| Hydroxypropylmethylcellulose, 4000 USP | 5,280 |
| Hydroxypropylmethylcellulose, 3 mPas | 22,000 |
| Triethylcitrat | 5,280 |
| Glycerol Ph.Eur./USP | 5,280 |
| Insulin glulisin | 12,000 |
| Na₂HPO₄ | 2,640 |
| Betacyclodextrin | 4,998 |
| Poloxamer 188 | 5,500 |
| Polyoxyethylene lauryl ether | 1,601 |
| Dinatriumedetat | 0,320 |
| Methyl-4-hydroxybenzoat | 0,114 |
| Salzsäure | 0,563 |
| Summe: | 100,000 |

Bei dem resultierenden zweischichtigen Film hatte die getrocknete Deckschicht ein Flächengewicht von 48,0 g/m² und die wirkstoffhaltige Schicht ein Flächengewicht von 114 g/m². Aus dem Film wurden Einzeldosen à 4,11 cm² ausgestanzt.

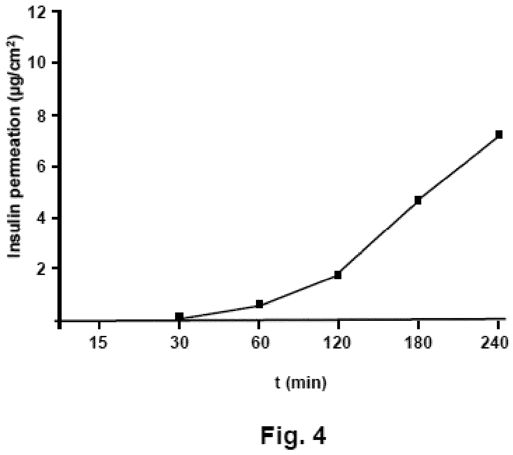

Die in einem in vitro-Permeationstest gemäß Beispiel 9 mit filmförmigen Darreichungsformen gemäß diesem Beispiel erzielbaren Permeationsraten sind in Fig. 4 graphisch dargestellt.

### Beispiel 8

Die Komponenten für die Deckschicht wurden in Ethanol (96 %-ig) gelöst und mit einem Filmziehgerat auf die vorstehend beschriebene wirkstoffhaltige Schicht aufgebracht. Das Laminat wurde anschließend bei einer Temperatur von 50 °C getrocknet.

Zusammensetzung der Deckschicht:

| Komponente | Anteil in Gew.-% |
|---|---|
| Ethylcellulose 100 | 93,500 |
| Triethylcitrat | 5,000 |
| Eisenoxid, gelb | 1,500 |
| Summe | 100,000 |

Die Komponenten für die wirkstoffhaltige Schicht wurden in einem Lösungsmittelgemisch aus 20 Gew.-% Ethanol und 80 Gew.-% Wasser gelöst und mit einem Filmziehgerat auf eine Polyethylenterephthalat-Folie aufgebracht und anschließend bei einer Temperatur von 50 °C getrocknet.

Bei dem resultierenden zweischichtigen Film hatte die getrocknete Deckschicht ein Flächengewicht von 48,0 g/m² und die wirkstoffhaltige Schicht ein Flächengewicht von 114 g/m². Aus dem Film wurden Einzeldosen à 4,11 cm² ausgestanzt.

Zusammensetzung der wirkstoffhaltigen Schicht

| Komponente | Anteil in Gew.-% |
|---|---|
| Hydroxypropylmethylcellulose, 100 USP | 30,000 |
| Hydroxypropylmethylcellulose, 4000 USP | 4,500 |
| Hydroxypropylmethylcellulose, 3 mPas | 18,370 |
| Glycerol Ph.Eur./USP | 20,000 |
| Insulin lispro | 15,000 |
| Citronensäure | 3,000 |
| Natriumhydroxid | 0,880 |
| Trinatriumcitrat | 5,800 |
| Trehalose | 1,000 |
| Betacyclodextrin | 1,000 |
| Dinatriumedetat | 0,350 |
| Methyl-4-hydroxybenzoat | 0,100 |
| Summe: | 100,000 |

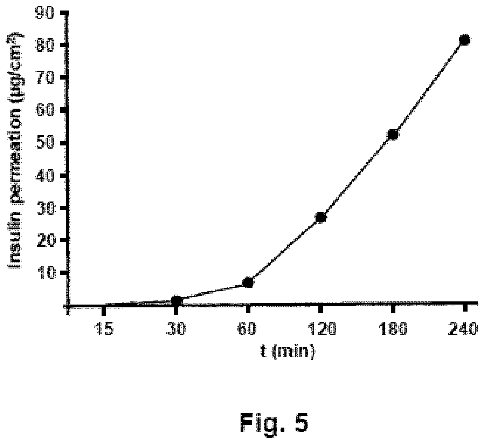

Die in einem in vitro-Permeationstest gemäß Beispiel 6 mit filmförmigen Darreichungsformen gemäß diesem Beispiel erzielbaren Permeationsraten sind in Fig. 5 graphisch dargestellt.

### Beispiel 9: Bestimmung der Permeation von Insulin/-analoga in vitro

Die Freisetzung und transmukosale Permeation von Insulin oder Insulinanaloga wurde mit Hilfe eines in vitro-Modells unter Verwendung des Models für menschliche Mundepithelien der Firma Skin Ethic, Lyon, FR) untersucht. Bei diesem Modell werden menschliche Keratinocyten der Zelllinie TR146 auf Polycarbonatfiltern kultiviert, so dass sich in einem chemisch definierten Medium ein Epithelgewebe an der Flüssigkeitsgrenzfläche zur Luft bildet, das kein Stratum corneum aufweist und somit zumindest histologisch der Mundschleimhaut entspricht.

Die einzelnen, das Modellgewebe aufweisenden Polycarbonatfilter wurden in einer Diffusionskammer eingesetzt, um Donor- und Akzeptorkompartiment voneinander zu trennen, wobei das Epithel zum Lumen des Donorkompartiments ausgerichtet war. Eine filmförmige Darreichungsform wurde auf das Epithel aufgebracht und in vorgegebenen Zeitintervallen wurde der Gehalt an Wirkstoff im Akzeptorkompartiment bestimmt. Die Ergebnisse zu den in den Beispielen 1 bis 4 beschriebenen Zubereitungen sind in den Figuren 1 und 2 graphisch dargestellt.

## Patentansprüche

1. Filmförmige Darreichungsform zur Verabreichung eines Peptid-Antidiabetikums über die Mundschleimhaut, umfassend mindestens eine wirkstoffhaltige Schicht, die mindestens ein Peptid-Antidiabetikum enthält und die ein Gemisch aus mindestens zwei Hydroxypropylmethylcellulosen umfasst, die sich in ihrer Viskosität voneinander unterscheiden.

2. Die filmförmige Darreichungsform gemäß Anspruch 1, wobei das Gemisch verschiedener Hydroxypropylmethylcellulosen ein Gemisch aus einer ersten Hydroxypropylmethylcellulose und einer zweiten Hydroxypropylmethylcellulose ist, bei der die erste Hydroxypropylmethylcellulose eine Hydroxypropylmethylcellulose mit hoher Viskosität und die zweite Hydroxypropylmethylcellulose eine Hydroxypropylmethylcellulose mit mittlerer Viskosität ist, wobei die Hydroxypropylmethylcellulose hoher Viskosität eine Viskosität von etwa 3.000 mPa·s bis etwa 5.600 mPa·s aufweist und wobei die Hydroxypropylmethylcellulose mittlerer Viskosität eine Viskosität von etwa 75 mPa·s bis etwa 140 mPa·s aufweist, jeweils gemessen mit einer 2 %-igen Lösung in Wasser bei einer Temperatur von 20 °C in einem Brookfield LV Viskosimeter.

3. Die filmförmige Darreichungsform gemäß Anspruch 1, wobei das Gemisch verschiedener Hydroxypropylmethylcellulosen ein Gemisch aus einer ersten Hydroxypropylmethylcellulose, einer zweiten Hydroxypropylmethylcellulose und einer dritten Hydroxypropylmethylcellulose ist, bei der die erste Hydroxypropylmethylcellulose eine Hydroxypropylmethylcellulose mit hoher Viskosität, die zweite Hydroxypropylmethylcellulose eine Hydroxypropylmethylcellulose mit mittlerer Viskosität und die dritte Hydroxypropylmethylcellulose eine Hydroxypropylmethylcellulose mit niedriger Viskosität ist, wobei die Hydroxypropylmethylcellulose hoher Viskosität eine Viskosität von etwa 3.000 mPa·s bis etwa 5.600 mPa·s aufweist, wobei die Hydroxypropylmethylcellulose mittlerer Viskosität eine Viskosität von etwa 75 mPa·s bis etwa 140 mPa·s aufweist und wobei die Hydroxypropylmethylcellulose niedriger Viskosität eine Viskosität von etwa 3 mPa·s aufweist, jeweils gemessen mit einer 2 %-igen Lösung in Wasser bei einer Temperatur von 20 °C in einem Brookfield LV Viskosimeter.

4. Die filmförmige Darreichungsform gemäß einem der Ansprüche 1 bis 3, wobei das mindestens eine Peptid-Antidiabetikum aus der Gruppe ausgewählt ist, die aus Insulinen, Insulin-Analoga, Inkretinen und Inkretin-Mimetika besteht.

5. Die filmförmige Darreichungsform gemäß einem der Ansprüche 1 bis 4, ferner aufweisend eine Deckschicht, vorzugsweise eine Deckschicht, die Ethylcellulose umfasst.

6. Die filmförmige Darreichungsform gemäß Anspruch 5, wobei der Gehalt an Ethylcellulose in der Deckschicht mindestens 50 Gew.-%, vorzugsweise mindestens 55 Gew.-%, besonders bevorzugt mindestens 60 Gew.-%, und ganz besonders bevorzugt mindestens 65 Gew.-%, jedoch maximal 99 Gew.-%, vorzugsweise maximal 95 Gew.-%, besonders bevorzugt maximal 94 Gew.-%, und ganz besonders bevorzugt maximal 93,5 Gew.-% beträgt, bezogen auf das Gesamtgewicht der Deckschicht.

7. Ein Verfahren zur Herstellung einer filmförmigen Darreichungsform zur transmukosalen Verabreichung eines Peptid-Antidiabetikums gemäß einem der Ansprüche 1 bis 6, umfassend die Schritte:
- Lösen und/oder Suspendieren der Komponenten für eine wirkstoffhaltige Schicht in einem Lösungsmittel,
- Aufbringen der Lösung oder Suspension auf einen Träger in definierter Dicke; und
- anschließendes Trocknen.

8. Das Verfahren gemäß Anspruch 7, ferner umfassend,
- Lösen und/oder Suspendieren der Komponenten für eine Deckschicht in einem Lösungsmittel,
- Aufbringen der Lösung oder Suspension für die Deckschicht auf einen Träger, und
- anschließendes Trocknen der Deckschicht.

9. Das Verfahren gemäß Anspruch 7 oder 8, wobei das Lösungsmittel für das Lösen oder Suspendieren der Komponenten für die wirkstoffhaltige Schicht und/oder für die Komponenten für die Deckschicht aus der Gruppe ausgewählt ist, die Wasser und Gemische aus Wasser und Ethanol umfasst, vorzugsweise ein Gemisch aus Wasser und Ethanol in einem Verhältnis von etwa 80 Vol.-% : 20 Vol.-% oder ein Gemisch aus Wasser und Ethanol in einem Verhältnis von etwa 4 Vol-% : 96 Vol-%.

10. Das Verfahren gemäß einem der Ansprüche 7 bis 9, wobei das Trocknen bei einer Temperatur von nicht mehr als 80 °C, vorzugsweise bei einer Temperatur von nicht mehr als 50 °C erfolgt.

11. Das Verfahren gemäß einem der Ansprüche 7 bis 10, wobei das Aufbringen der Lösung oder Suspension mit den Komponenten für die wirkstoffhaltige Schicht auf eine vorgefertigte wirkstoffhaltige Schicht oder eine vorgefertigte Deckschicht erfolgt.

12. Das Verfahren gemäß einem der Ansprüche 7 bis 11, ferner umfassend das Vereinzeln der Darreichungsform oder das Schneiden in Streifen vorbestimmter Breite und Länge; und nachfolgendes Verpacken.

## Claims

1. A film-like dosage form for administering an antidiabetic peptide via the oral mucosa, comprising at least one layer containing active ingredient which contains at least one antidiabetic peptide and which comprises a mixture of at least two hydroxypropyl methylcelluloses which differ in viscosity from each other.

2. The film-like dosage form according to claim 1, wherein the mixture of different hydroxypropyl methylcelluloses is a mixture of a first hydroxypropyl methylcellulose and a second hydroxypropyl methylcellulose in which the first hydroxypropyl methylcellulose is a hydroxypropyl methylcellulose of high viscosity and the second hydroxypropyl methylcellulose is a hydroxypropyl methylcellulose of medium viscosity, wherein the hydroxypropyl methylcellulose of high viscosity has a viscosity of approximately 3,000 mPa·s to approximately 5,600 mPa·s and wherein the hydroxypropyl methylcellulose of medium viscosity has a viscosity of approximately 75 mPa·s to approximately 140 mPa·s, measured in each case with a 2%-strength solution in water at a temperature of 20°C in a Brookfield LV viscometer.

3. The film-like dosage form according to claim 1, wherein the mixture of different hydroxypropyl methylcelluloses is a mixture of a first hydroxypropyl methylcellulose, a second hydroxypropyl methylcellulose and a third hydroxypropyl methylcellulose in which the first hydroxypropyl methylcellulose is a hydroxypropyl methylcellulose of high viscosity, the second hydroxypropyl methylcellulose is a hydroxypropyl methylcellulose of medium viscosity and the third hydroxypropyl methylcellulose is a hydroxypropyl methylcellulose of low viscosity, wherein the hydroxypropyl methylcellulose of high viscosity has a viscosity of approximately 3,000 mPa·s to approximately 5,600 mPa·s, wherein the hydroxypropyl methylcellulose of medium viscosity has a viscosity of approximately 75 mPa·s to approximately 140 mPa·s and wherein the hydroxypropyl methylcellulose of low viscosity has a viscosity of approximately 3 mPa·s, measured in each case with a 2%-strength solution in water at a temperature of 20°C in a Brookfield LV viscometer.

4. The film-like dosage form according to one of claims 1 to 3, wherein the at least one antidiabetic peptide is selected from the group which consists of insulins, insulin analogues, incretins and incretin mimetics.

5. The film-like dosage form according to one of claims 1 to 4, further having a cover layer, preferably a cover layer which comprises ethylcellulose.

6. The film-like dosage form according to claim 5, wherein the content of ethylcellulose in the cover layer amounts to at least 50 wt.%, preferably at least 55 wt.%, especially preferably at least 60 wt.%, and very especially preferably at least 65 wt.%, but at most 99 wt.%, preferably at most 95 wt.%, especially preferably at most 94 wt.%, and very especially preferably at most 93.5 wt.%, relative to the total weight of the cover layer.

7. A method for producing a film-like dosage form for transmucosal administration of an antidiabetic peptide according to one of claims 1 to 6, comprising the steps:
- dissolving and/or suspending the components for a layer containing active ingredient in a solvent,
- applying the solution or suspension to a support in a defined thickness; and
- subsequent drying.

8. The method according to claim 7, further comprising,
- dissolving and/or suspending the components for a cover layer in a solvent,
- applying the solution or suspension for the cover layer to a support, and
- subsequently drying the cover layer.

9. The method according to claim 7 or Claim 8, wherein the solvent for dissolving or suspending the components for the layer containing active ingredient and/or for the components for the cover layer is selected from the group comprising water and mixtures of water and ethanol, preferably a mixture of water and ethanol in a ratio of approximately 80 vol.% : 20 vol.% or a mixture of water and ethanol in a ratio of approximately 4 vol.% : 96 vol.%.

10. The method according to one of claims 7 to 9, wherein the drying takes place at a temperature of not greater than 80°C, preferably at a temperature of not greater than 50°C.

11. The method according to one of claims 7 to 10, wherein the applying of the solution or suspension with the components for the layer containing active ingredient takes place on a prefabricated layer containing active ingredient or a prefabricated cover layer.

12. The method according to one of claims 7 to 11, further comprising separating the dosage form or cutting into strips of predetermined width and length; and subsequent packaging.

## Revendications

1. Forme pharmaceutique sous forme de film pour l'administration d'un antidiabétique peptidique par la muqueuse buccale, comprenant au moins une couche contenant un principe actif, qui contient au moins un antidiabétique peptidique et qui comprend un mélange d'au moins deux hydroxypropylméthylcelluloses, qui se différencient l'une de l'autre par leur viscosité.

2. Forme pharmaceutique sous forme de film selon la revendication 1, dans laquelle le mélange de différentes hydroxypropylméthylcelluloses est un mélange d'une première hydroxypropylméthylcellulose et d'une deuxième hydroxypropylméthylcellulose, pour laquelle la première hydroxypropylméthylcellulose est une hydroxypropylméthylcellulose avec une viscosité élevée et la deuxième hydroxypropylméthylcellulose est une hydroxypropylméthylcellulose avec une viscosité moyenne, dans laquelle l'hydroxypropylméthylcellulose de viscosité élevée présente une viscosité de sensiblement 3 000 mPa·s à sensiblement 5 600 mPa·s et dans laquelle l'hydroxypropylméthylcellulose de viscosité moyenne présente une viscosité de sensiblement 75 mPa·s jusqu'à sensiblement 140 mPa·s, respectivement mesurées avec une solution à 2 % dans l'eau à une température de 20 °C dans un viscosimètre Brookfield LV.

3. Forme pharmaceutique sous forme de film selon la revendication 1, dans laquelle le mélange de différentes hydroxypropylméthylcelluloses est un mélange d'une première hydroxypropylméthylcellulose, d'une deuxième hydroxypropylméthylcellulose et d'une troisième hydroxypropylméthylcellulose, pour laquelle la première hydroxypropylméthylcellulose est une hydroxypropylméthylcellulose avec une viscosité élevée, la deuxième hydroxypropylméthylcellulose une hydroxypropylméthylcellulose avec une viscosité moyenne et la troisième hydroxypropylméthylcellulose une hydroxypropylméthylcellulose avec une faible viscosité, dans laquelle l'hydroxypropylméthylcellulose de viscosité élevée présente une viscosité de sensiblement 3 000 mPa·s à sensiblement 5 600 mPa·s, dans laquelle l'hydroxypropylméthylcellulose de viscosité moyenne présente une viscosité de sensiblement 75 mPa·s à sensiblement 140 mPa·s et dans laquelle l'hydroxypropylméthylcellulose de faible viscosité présente une viscosité de sensiblement 3 mPa·s, respectivement mesurées avec une solution à 2 % dans l'eau à une température de 20 °C dans un viscosimètre Brookfield LV.

4. Forme pharmaceutique sous forme de film selon l'une quelconque des revendications 1 à 3, dans laquelle l'au moins un antidiabétique peptidique est choisi dans le groupe qui est constitué des insulines, analogues de l'insuline, incrétines et incrétinomimétiques.

5. Forme pharmaceutique sous forme de film selon l'une quelconque des revendications 1 à 4, présentant en outre une couche de recouvrement, de préférence une couche de recouvrement qui comprend de l'éthylcellulose.

6. Forme pharmaceutique sous forme de film selon la revendication 5, dans laquelle la teneur en éthylcellulose dans la couche de recouvrement atteint au moins 50 % en poids, de préférence au moins 55 % en poids, de manière particulièrement préférée au moins 60 % en poids et de manière préférée entre toutes au moins 65 % en poids, mais au maximum 99 % en poids, de préférence au maximum 95 % en poids, de manière particulièrement préférée au maximum 94 % en poids, et de manière préférée entre toutes au maximum 93,5 % en poids, rapporté au poids total de la couche de recouvrement.

7. Procédé pour fabriquer une forme pharmaceutique sous forme de film pour l'administration par voie transmuqueuse d'un antidiabétique peptidique selon l'une quelconque des revendications 1 à 6, comprenant les étapes :
- de dissolution et/ou mise en suspension des composants pour une couche contenant un principe actif dans un solvant,
- d'application de la solution ou suspension sur un support d'épaisseur définie ; et
- de séchage subséquent.

8. Procédé selon la revendication 7, comprenant en outre
- la dissolution et/ou mise en suspension des composants pour une couche de recouvrement dans un solvant,
- l'application de la solution ou suspension pour la couche de recouvrement sur un support, et
- le séchage subséquent de la couche de recouvrement.

9. Procédé selon la revendication 7 ou 8, dans lequel le solvant pour la solution ou mise en suspension des composants pour la couche contenant un principe actif et/ou pour les composants pour la couche de recouvrement est choisi dans le groupe qui comporte de l'eau et des mélanges d'eau et d'éthanol, de préférence un mélange d'eau et d'éthanol dans un rapport de sensiblement 80 %vol:20 %vol ou un mélange d'eau et d'éthanol dans un rapport de sensiblement 4 %vol:96 %vol.

10. Procédé selon l'une quelconque des revendications 7 à 9, dans lequel le séchage s'effectue à une température non supérieure à 80 °C, de préférence à une température non supérieure à 50 °C.

11. Procédé selon l'une quelconque des revendications 7 à 10, dans lequel l'application de la solution ou suspension avec les composants pour la couche contenant un principe actif s'effectue sur une couche contenant un principe actif préfabriquée ou une couche de recouvrement préfabriquée.

12. Procédé selon l'une quelconque des revendications 7 à 11, comprenant en outre la séparation de la forme pharmaceutique ou le découpage en bandes de largeur et longueur prédéfinie ; et l'emballage subséquent.
